# EUROPEAN PATENT APPLICATION

(11) **EP 4 279 090 A1**
(43) Date of publication of application: **22.11.2023**
(21) Application number: 22174718.1
(22) Date of filing: 20.05.2022
(51) Int. Cl.: A61K 47/68, A61K 47/69, A61K 9/00, A61K 9/19, A61K 47/18, A61K 47/26, A61P 35/00

(54) **COMPOSITION COMPRISING A PYRROLOBENZODIAZEPINE-BASED ANTIBODY DRUG CONJUGATE**

(71) Applicant: ADC Therapeutics SA, 1066 Epalinges (CH); MedImmune Limited, Cambridge Cambridgeshire CB21 6GH (GB)
(72) Inventor: The designation of the inventor has not yet been filed
(74) Representative: Finnie, Nicholas James

(57) **Abstract**

This disclosure relates to pharmaceutical formulations comprising pyrrolobenzodiazepine-based antibody-drug conjugates (ADCs) and having a pH of at least 6.2. In one embodiment, the ADC is selected from ADCT-601 (mipasetamab uzoptirine) and ADCT-701.

## Description

### Field

The present disclosure relates to novel formulations of pyrrolobenzodiazepine-based antibody drug conjugates (ADCs).

### Background

AXL is member of the human TAM family of receptor tyrosine kinases. The Gas6/AXL signalling pathway is associated with tumour cell growth, metastasis, invasion, epithelial-mesenchymal transition (EMT), angiogenesis, drug resistance, immune regulation and stem cell maintenance. Increased AXL expression has been associated with numerous cancers including lung cancer, breast cancer, pancreatic cancer, ovarian cancer, colon cancer and melanoma among others. Different therapeutic agents targeting AXL have been developed, typically including small molecule inhibitors, monoclonal antibodies (mAbs), nucleotide aptamers, soluble receptors, and several natural compounds.

ADCT-601 is a novel therapy based on the use of antibody drug conjugates comprising as the therapeutic payload, a pyrrolobenzodiazepine dimer (PBD).

### Summary

The present disclosure is directed to pharmaceutical compositions comprising antibody-drug conjugates comprising particular drug-linkers based on certain PBDs. We have developed formulations that improve the stability of the PBD payload whilst maintaining a good level of solubility needed for efficient manufacturing.

Accordingly in a first aspect, the present disclosure relates to a pharmaceutical composition comprising an antibody drug conjugate (ADC) of formula:

Ab-(L-D)ₚ

wherein:
Ab is an antibody;
(L-D) is a drug-linker which comprises a PBD dimer, and which has the following formula (I): 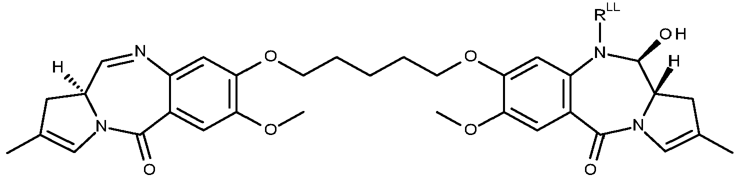
where R^{LL} is a linker for attachment to an antibody;
and p is a value from about 1 to about 8;
wherein the pharmaceutical composition has a pH of at least about 6.2.

In one embodiment, the linker comprises (typically in the following order from the drug to the end proximal the antibody): (i) a cleavable linker, such as Val-Ala or Val-Cit, together with a self-immolative moiety such as PABA or PABC attached to the drug; (ii) a spacer such as PEG; (iii) optionally a hydrophilic spacer moiety such as: 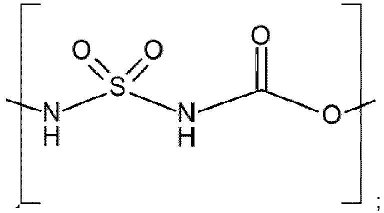 and (iv) a click chemistry reaction product, such as: 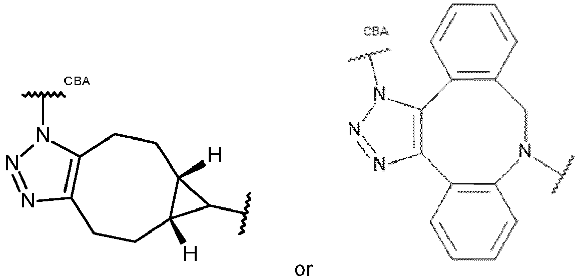 (wherein 'CBA' is Ab).

In a specific embodiment, L-D is:

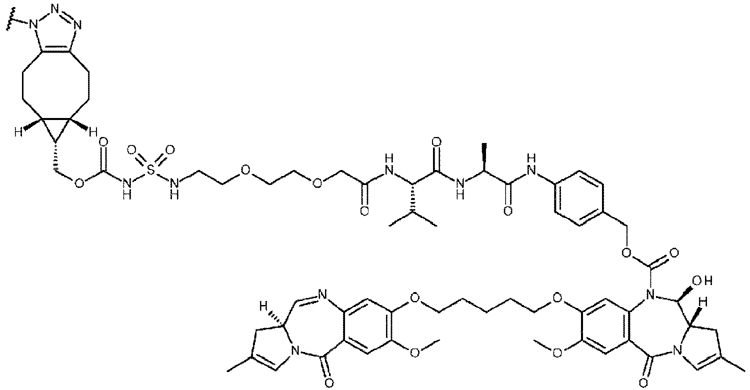

In a specific embodiment the pharmaceutical composition has a pH of from about 6.2 to about 7.0, such as from about 6.3 to about 6.9 or from about 6.4 to about 6.7.

The pharmaceutical composition may comprise a buffering agent, such as histidine/histidine HCI.

In one embodiment the concentration of the buffering agent, such as histidine/histidine HCI, is from about 10 to about 30 mM, such as about 20 mM.

The pharmaceutical composition may comprise a surfactant, such as polysorbate 20.

In one embodiment the concentration of the surfactant, such as polysorbate 20, is from about 0.01 to 0.1% w/v.

The pharmaceutical composition may comprise a sugar, such as sucrose.

In one embodiment the concentration of the sugar, such as sucrose, is from about 100 mM to about 250 mM, such as about 175 mM.

The ADC may be at a concentration of about 5 mg/ml to about 60 mg/ml, about 5 mg/ml to about 50 mg/ml, about 5 mg/ml to about 40 mg/ml, about 5 mg/ml to about 5 mg/ml, or about 5 mg/ml to about 20 mg/ml.

In a specific embodiment, the Ab is an anti-AXL antibody, such as an anti-AXL antibody which comprises a heavy chain and a light chain, wherein (i) the heavy chain comprises a VH domain having a VH CDR1 with the amino acid sequence of SEQ ID NO.3; a VH CDR2 with the amino acid sequence of SEQ ID NO.4; and a VH CDR3 with the amino acid sequence of SEQ ID NO.5; and (ii) the light chain comprises a VL domain having a VL CDR1 with the amino acid sequence of SEQ ID NO.6; a VL CDR2 with the amino acid sequence of SEQ ID NO.7, and a VL CDR3 with the amino acid sequence of SEQ ID NO.8.

In a second aspect, the present disclosure relates to a lyophilized pharmaceutical composition produced by lyophilization of a pharmaceutical composition of the first aspect which is in liquid form.

In a related aspect is the pharmaceutical composition of the first aspect has been reconstituted from a lyophilized pharmaceutical composition of the second aspect with a diluent such as sterile water for injection (SWFI) to produce a reconstituted liquid composition.

In one embodiment, the ADC is selected from ADCT-601 (mipasetamab uzoptirine) and ADCT-701.

The pharmaceutical compositions disclosed herein may be administered to a subject in need thereof. Accordingly the present disclosure also relates to a method of treating an subject suffering from a proliferative disorder, such as cancer, by administered a pharmaceutical compositions as disclosed herein to the subject, such as a human subject.

The present disclosure also relates to a pharmaceutical compositions as disclosed herein for use in therapy, such as in treating a proliferative disorder in a subject, as well as the use of a pharmaceutical compositions as disclosed herein in the manufacture of a medicament for use in in treating a proliferative disorder in a subject.

### Detailed Description

The formulations described herein comprise, as an active ingredient, an antibody drug conjugate comprising an antibody and a drug-linker which includes a pyrrolobenzodiazepine dimer.

The term "about" in relation to a value or parameter encompasses an error range of any one of ± 5%, ±4%, ±3%, ±2%, or ±1% of the stated value or parameter, including any range in between these values.

### Antibodies

The term "antibody" herein is used in the broadest sense and specifically covers monoclonal antibodies, including both intact antibodies and antibody fragments, so long as they exhibit the desired biological activity, for example, the ability to bind a tumour antigen. Antibodies may be murine, rat, human, humanized, chimeric, or derived from other species. An antibody includes a full-length immunoglobulin molecule or an immunologically active portion of a full-length immunoglobulin molecule, i.e., a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g. IgG, IgE, IgM, IgD, and IgA), class (e.g. IgG1, IgG2, IgG3, IgG4, IgA1 and IgA2) or subclass, or allotype (e.g. human G1m1, G1m2, G1m3, non-G1m1 [that, is any allotype other than G1m1], G1m17, G2m23, G3m21, G3m28, G3m11, G3m5, G3m13, G3m14, G3m10, G3m15, G3m16, G3m6, G3m24, G3m26, G3m27, A2m1, A2m2, Km1, Km2 and Km3) of immunoglobulin molecule.

A variety of immunoglobulin variant formats are known in the art which are derived from conventional immunoglobulins, such as bispecific antibodies, scFvs, nanobodies and the like. These are all within the scope of the term "antibody" provided they retain the desired biological activity, for example, the ability to bind a tumour antigen.

### Preferred antibodies

Typically, the antibody targets a cell surface antigen, such as a tumour-associated antigen (e.g. an antigen whose expression is up-regulated in a tumour cell).

In some embodiments, the antibody binds AXL. In some embodiments, the AXL polypeptide corresponds to Genbank accession no. AAH32229, version no. AAH32229.1 GI:21619004, record update date: March 6, 2012 01:18 PM. In one embodiment, the nucleic acid encoding AXL polypeptide corresponds to Genbank accession no. M76125, version no. M76125.1 GI:292869, record update date: Jun 23, 2010 08:53 AM. In some embodiments the antibody comprises a VH domain having a VH CDR1 with the amino acid sequence of SEQ ID NO.5, a VH CDR2 with the amino acid sequence of SEQ ID NO.6, and a VH CDR3 with the amino acid sequence of SEQ ID NO.7. The antibody may further comprise a VL domain having a VL CDR1 with the amino acid sequence of SEQ ID NO.8, a VL CDR2 with the amino acid sequence of SEQ ID NO.9, and a VL CDR3 with the amino acid sequence of SEQ ID NO.10. In preferred embodiments the antibody comprises a VH domain having the sequence of SEQ ID NO.1 and a VL domain having the sequence of SEQ ID NO.2.

In some embodiments, the antibody binds DLK-1. In some embodiments, the DLK1 polypeptide corresponds to Genbank accession no. CAA78163, version no. CAA78163.1, record update date: Feb 2, 2011 10:34 AM. In one embodiment, the nucleic acid encoding DLK1 polypeptide corresponds to Genbank accession no. Z12172, version no Z12172.1, record update date: Feb 2, 2011 10:34 AM. The antibody may comprise a VH domain having a VH CDR1 with the amino acid sequence of SEQ ID NO.15, a VH CDR2 with the amino acid sequence of SEQ ID NO.16, and a VH CDR3 with the amino acid sequence of SEQ ID NO.17. The antibody may further comprise a VL domain having a VL CDR1 with the amino acid sequence of SEQ ID NO.18, a VL CDR2 with the amino acid sequence of SEQ ID NO.19, and a VL CDR3 with the amino acid sequence of SEQ ID NO.20. Preferably the antibody comprises a VH domain having the sequence of SEQ ID NO.11 and a VL domain having the sequence of SEQ ID NO.12. In some embodiments the antibody comprises a heavy chain having the sequence of SEQ ID NO. 13 or 15 paired with a light chain having the sequence of SEQ ID NO.14.

As used herein to describe antibodies, "binds [antigen]" (e.g. "binds AXL") means that the antibody binds the antigen with a higher affinity than a non-specific partner such as Bovine Serum Albumin (BSA, Genbank accession no. CAA76847, version no. CAA76847.1 GI:3336842, record update date: Jan 7, 2011 02:30 PM). In some embodiments the antibody binds the antigen with an association constant (Kₐ) at least 2, 3, 4, 5, 10, 20, 50, 100, 200, 500, 1000, 2000, 5000, 10⁴, 10⁵ or 10⁶-fold higher than the antibody's association constant for BSA, when measured at physiological conditions. The antibodies of the disclosure can bind the antigen with a high affinity. For example, in some embodiments the antibody can bind the antigen with a K_{D} equal to or less than about 10⁻⁶ M, such as equal to or less than one of 1 x 10⁻⁶, 10⁻⁷, 10⁻⁸, 10-⁹,10-¹⁰, 10⁻¹¹, 10⁻¹², 10⁻¹³ or 10⁻¹⁴.

"Antibody fragments" comprise a portion of a full length antibody, generally the antigen binding or variable region thereof. Examples of antibody fragments include Fab, Fab', F(ab')₂, and scFv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

Typically the antibody is a monoclonal antibody, which herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US 4,816,567; and Morrison et al. (1984) Proc. Natl. Acad. Sci. USA, 81:6851-6855). Chimeric antibodies include "primatized" antibodies comprising variable domain antigen-binding sequences derived from a non-human primate (e.g. Old World Monkey or Ape) and human constant region sequences.

An "intact antibody" herein is one comprising VL and VH domains, as well as a light chain constant domain (CL) and heavy chain constant domains, CH1, CH2 and CH3. The constant domains may be native sequence constant domains (e.g. human native sequence constant domains) or amino acid sequence variant thereof. The intact antibody may have one or more "effector functions" which refer to those biological activities attributable to the Fc region (a native sequence Fc region or amino acid sequence variant Fc region) of an antibody. Examples of antibody effector functions include C1q binding; complement dependent cytotoxicity; Fc receptor binding; antibody-dependent cell-mediated cytotoxicity (ADCC); phagocytosis; and down regulation of cell surface receptors such as B cell receptor and BCR.

### PBD Cytotoxin

Pyrrolobenzodiazepines (PBDs) suitable for use in the present disclosure in some embodiments have the ability to recognise and bond to specific sequences of DNA; the preferred sequence is PuGPu. PBDs are of the general structure:

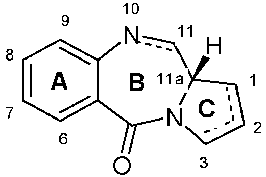

They differ in the number, type and position of substituents, in both their aromatic A rings and pyrrolo C rings, and in the degree of saturation of the C ring. In the B-ring there is either an imine (N=C), a carbinolamine(NH-CH(OH)), or a carbinolamine methyl ether (NH-CH(OMe)) at the N10-C11 position which is the electrophilic centre responsible for alkylating DNA. All of the known natural products have an (S)-configuration at the chiral C11a position which provides them with a right-handed twist when viewed from the C ring towards the A ring. This gives them the appropriate three-dimensional shape for isohelicity with the minor groove of B-form DNA, leading to a snug fit at the binding site (Kohn, In Antibiotics III. Springer-Verlag, New York, pp. 3-11 (1975); Hurley and Needham-VanDevanter, Acc. Chem. Res., 19, 230-237 (1986)). Their ability to form an adduct in the minor groove, enables them to interfere with DNA processing, hence their use as antitumour agents.

The cytotoxin is typically a PBD dimer, which has the following formula (I):

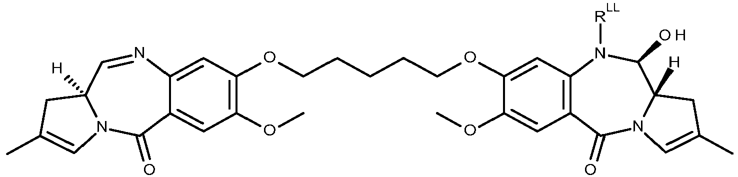

Where R^{LL} is a linker for attachment to an antibody. When released from the linker R^{LL}, the bond between N10, to which R^{LL} was originally attached, and C11 typically together form a double bond between the nitrogen and carbon atoms to which they are attached (an imine (C=N)).

### Drug-linkers

The linker typically comprises a (i) moiety involved in conjugation to the antibody at a site of interest, (ii) one or more spacers that mask the hydrophobicity of the cytotoxin payload, reduce cellular efflux mechanisms and/or increase overall stability, such as a polyethylene glycol (PEG) chain within the linker or a polar functional group such as a sulphonyl; (iii) a cleavable moiety; and (iv) a self-immolative moiety to enable release of the final drug payload without any linker components remaining.

The functionality that allows conjugation to the cell binding agent is based on the site of conjugation and its chemistry. N-hydroxysuccinimide esters are a common choice for functionalizing amines, especially when coupling to ε-lysine residues. For conjugation to cysteines, thiol-reactive maleimide is the most applied reactivehandle, although it is also possible to create a disulfide bridge by oxidation with a linker bearing a sulfhydryl group. Aldehyde or keto functional groups such as oxidized sugar groups or pAcPhe unnatural amino acids can be reacted with hydrazides and alkoxyamines to yield acid-labile hydrazones or oxime bonds. In addition, a hydrazine can be coupled with an aldehyde via HIPS ligation to generate a stable C-C linkage.

More recent approaches have been based on the N-linked glycosylation site in antibodies, such as Asn-297 in IgG molecules. GlycoConnect^{™} (Synaffix), uses enzymes to trim the N-linked glycans to a GlcNAc core and then a further enzymatic process to introduce an activated moiety comprising azide which can then be used to incorporate the drug-linker using copper-free click chemistry.

In some embodiments, the antibody drug conjugates of the disclosure can be described as Ab-L-D, e.g. where Ab is an antibody, D is the PBD-containing cytotoxin and L is a linker. The number of Drug moieties per Ab (the drug loading, p) depends on the number of linkers attached to each Ab, and the number of Drug moieties per linker. Typically the drug loading, p, is from 1 to 8, such as from 1 to 4 or 2 to 4. Drug loading is typically considered on an average basis since variations can arise from the conjugation process. Methods for determining average drug loading are known in the art.

In one embodiment the linker (e.g. shown as R^{LL} in formula (I)) is of formula (Ia): 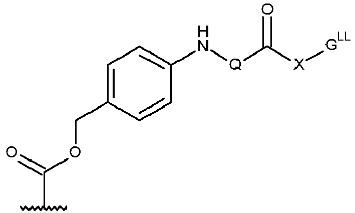 wherein
Q is: 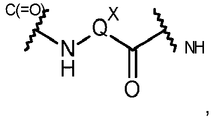 where Q^{X} is such that Q is an amino-acid residue, a dipeptide residue, a tripeptide residue or a tetrapeptide residue;

### X

X is: 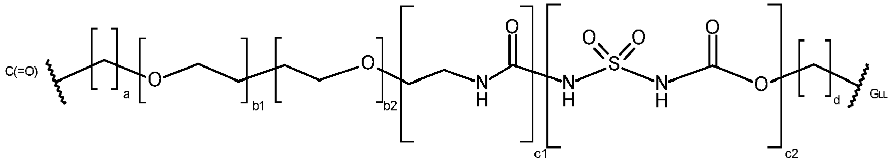 where a = 0 to 5, b1 = 0 to 16, b2 = 0 to 16, c1 = 0 or 1, c2 = 0 or 1, d = 0 to 5, wherein at least b1 or b2 = 0 and at least c1 or c2 = 0.

a may be 0, 1, 2, 3, 4 or 5. In some embodiments, a is 0 to 3. In some of these embodiments, a is 0 or 1. In further embodiments, a is 0. In further embodiments, a is 1.

b1 may be 0, 1, 2, 3, 4, 5, 6, 7, 8, 9, 10, 11, 12, 13, 14, 15 or 16. In some embodiments, b1 is 0 to 12. In some of these embodiments, b1 is 0 to 8, and may be 0, 2, 3, 4, 5 or 8. In further embodiments, b1 is 2.

b2 may be 0, 1, 2, 3, 4, 5, 6, 7, 8, 9, 10, 11, 12, 13, 14, 15 or 16. In some embodiments, b2 is 0 to 12. In some of these embodiments, b2 is 0 to 8, and may be 0, 2, 3, 4, 5 or 8. In further embodiments, b2 is 8.

Only one of b1 and b2 may not be 0.

c1 may be 0 or 1. c2 may be 0 or 1. Only one of c1 and c2 may not be 0.

d may be 0, 1, 2, 3, 4 or 5. In some embodiments, d is 0 to 3. In some of these embodiments, d is 1 or 2. In further embodiments, d is 2. In further embodiments, d is 5.

In one embodiment, c2 = 1 and c1 = 0.

In some embodiments of X, a is 1, b2 is 0, c1 is 0, c2 is 1, d is 2, and b1 may be from 0 to 8. In some of these embodiments, b1 is 0, 2, 3, 4, 5 or 8. In further embodiments, b1 is 2.

### Q^{X}

In one embodiment, Q is an amino acid residue. The amino acid may be a natural amino acid or a non-natural amino acid.

In one embodiment, Q is selected from: Phe, Lys, Val, Ala, Cit, Leu, Ile, Arg, and Trp, where Cit is citrulline.

In one embodiment, Q comprises a dipeptide residue. The amino acids in the dipeptide may be any combination of natural amino acids and non-natural amino acids. In some embodiments, the dipeptide comprises natural amino acids. Where the linker is a cathepsin labile linker, the dipeptide is the site of action for cathepsin-mediated cleavage. The dipeptide then is a recognition site for cathepsin.

In one embodiment, Q is selected from:
^{NH}-Phe-Lys-^{C=O}, ^{NH}-Val-Ala-^{C=O}, ^{NH}-Val-Lys-^{C=O}, ^{NH}-Ala-Lys-^{C=O}, ^{NH}-Val-Cit-^{C=O},
^{NH}-Phe-Cit-^{C=O}, ^{NH}-Leu-Cit-^{C=O}, ^{NH}-Ile-Cit-^{C=O}, ^{NH}-Phe-Arg-^{C=O}, ^{NH}-Trp-Cit-^{C=O}, and
^{NH}-Gly-Val- ^{C=O};
where Cit is citrulline.

Preferably, Q is selected from:
^{NH}-Phe-Lys-^{C=O}, ^{NH}-Val-Ala-^{C=O}, ^{NH}-Val-Lys-^{C=O}, ^{NH}-Ala-Lys-^{C=O}, and ^{NH}-Val-Cit-^{C=O}.

Most preferably, Q is selected from ^{NH}-Phe-Lys-^{C=O}, ^{NH}-Val-Cit-^{C=O} or ^{NH}-Val-Ala-^{C=O}.

Other dipeptide combinations of interest include:
^{NH}-Gly-Gly- ^{C=O}, ^{NH}-Gly-Val- ^{C=O}, ^{NH}-Pro-Pro- ^{C=O}, and ^{NH}-Val-Glu- ^{C=O}.

Other dipeptide combinations may be used, including those described by Dubowchik et al., Bioconjugate Chemistry, 2002, 13,855-869, which is incorporated herein by reference.

In some embodiments, Q is a tripeptide residue. The amino acids in the tripeptide may be any combination of natural amino acids and non-natural amino acids. In some embodiments, the tripeptide comprises natural amino acids. Where the linker is a cathepsin labile linker, the tripeptide is the site of action for cathepsin-mediated cleavage. The tripeptide then is a recognition site for cathepsin.

### G^{LL}

G^{LL} may be selected from:

| | | | |
|---|---|---|---|
| (G^{LL1-1}) | 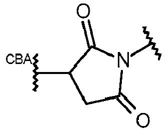 | (G^{LL8-1}) | 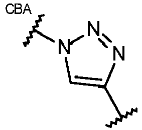 |
| (G^{LL1-2}) | 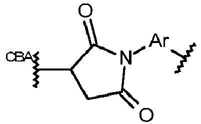 | (G^{LL8-2}) | 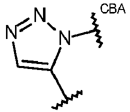 |
| (G^{LL2}) | 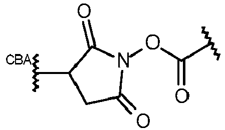 | (G^{LL9-1}) | 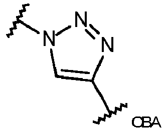 |
| (G^{LL3-1}) | 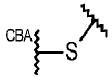 | (G^{LL9-2}) | 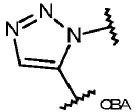 |
| (G^{LL3-2}) | 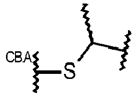 | (G^{LL10}) | 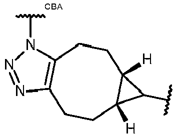 |
| (G^{LL-4}) | 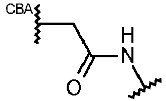 | (G^{LL11}) | 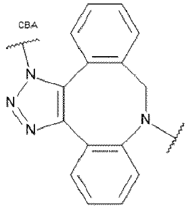 |
| (G^{LL5}) | 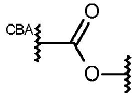 | (G^{LL12}) | 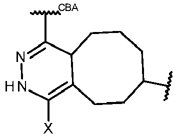 |
| (G^{LL6}) | 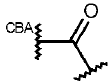 | (G^{LL13}) | 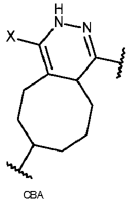 |
| (G^{LL7}) | 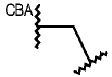 | (G^{LL14}) | 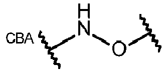 |

where Ar represents a C₅₋₆ arylene group, e.g. phenylene and X represents C₁₋₄ alkyl. CBA indicates the end of G^{LL} connected to the antibody.

C₅₋₆ arylene: The term "C₅₋₆ arylene", as used herein, pertains to a divalent moiety obtained by removing two hydrogen atoms from an aromatic ring atom of an aromatic compound. In this context, the prefixes (e.g. C₅₋₆) denote the number of ring atoms, or range of number of ring atoms, whether carbon atoms or heteroatoms. The ring atoms may be all carbon atoms, as in "carboarylene groups", in which case the group is phenylene (C₆). Alternatively, the ring atoms may include one or more heteroatoms, as in "heteroarylene groups".

In a particular embodiment, G^{LL} is selected from G^{LL10} and G^{LL11} , such as G^{LL} is G^{LL10}.

C₁₋₄ alkyl: The term "C₁₋₄ alkyl" as used herein, pertains to a monovalent moiety obtained by removing a hydrogen atom from a carbon atom of a hydrocarbon compound having from 1 to 4 carbon atoms, which may be aliphatic or alicyclic, and which may be saturated or unsaturated (e.g. partially unsaturated, fully unsaturated). The term "C₁₋ₙ alkyl" as used herein, pertains to a monovalent moiety obtained by removing a hydrogen atom from a carbon atom of a hydrocarbon compound having from 1 to n carbon atoms, which may be aliphatic or alicyclic, and which may be saturated or unsaturated (e.g. partially unsaturated, fully unsaturated). Thus, the term "alkyl" includes the sub-classes alkenyl, alkynyl, cycloalkyl, etc., discussed below.

The linker is typically connected to the PBD dimervia the N10 position, such as is shown in Formula I.

In a preferred embodiment the drug linker is:

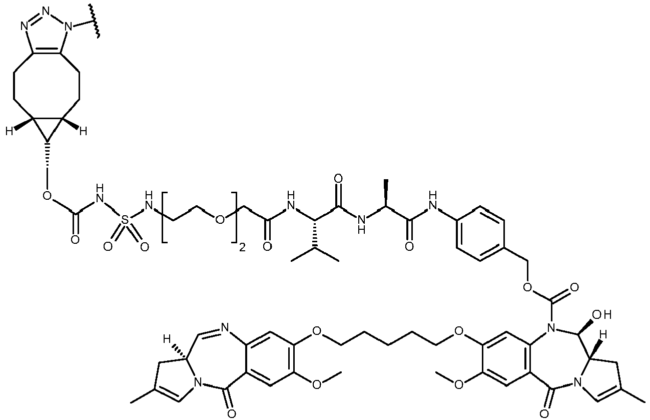

### Site of Conjugation and Drug Loading

Drug-linkers can be conjugated to a cell binding agent, such as an antibody, using a variety of methods known in the art and at a number of different sites. Conjugation sites include cysteine residues and lysine residues in the antibody sequence (endogenous or engineered), as well as sites of N-linked glycosylation following trimming (e.g. the GlycoConnect^{™} or GlyClick^{™} approaches). Thus in one embodiment the drug-linker is conjugated via a trimmed Asn-GlcNAc residue, typically at the endogenous N-linked glycosylation site in the antibody (Asn-297). Depending on the nature of the glycan, a suitable endoglycosidase may be selected to perform the trimming. The endoglycosidase is preferably selected from the group consisting of Endo S, Endo A, Endo F, Endo M, Endo D and Endo H enzymes and/or a combination thereof, the selection of which depends on the nature of the glycan. In a particular embodiment, as used in GlycoConnect, the GlcNAc is then reacted with a sugar derivative in the presence of a β-1-4-galactosyltransferase (GaIT). This is typically used to add a sugar moiety as well as a reactive group which is suitable for a further step where further linker components are added, such as a drug-linker, by a reaction with the reactive group.

The sugar derivative typically comprises a functional group that is a moiety comprising a bioorthogonal-reaction compatible reactive group, for example an unprotected or protected thiol, epoxide, maleimide, haloacetamide, o-phoshenearomatic ester, azide, fulminate, sulfonate ester, alkyne, cyanide, amino-thiol, carbonyl, aldehyde, generally any group capable of oxime and hydrazine formation, 1,2,4,5-tetrazine, norbornene, other stained or otherwise electronically activated alkene, a substituted or unsubstituted cycloalkyne, generally any reactive groups which form via bioorthogonal cycloaddition reaction a 1,3- or 1,5-disubstituted triazole, any diene or strained alkene dienophile that can react via inverse electron demand Diels-Alder (IEDDA) reaction, a protected or unprotected amine, a carboxylic acid, an aldehyde, an oxyamine.

In one embodiment, the reactive group is a group having a terminal azide. This group can, for example, react with a substituted or unsubstituted cycloalkyne, such as a substituted or unsubstituted heterocyclic strained alkyne, such as a DBCO (dibenzycyclooctyl) group or a BCN (bicyclononyne) group. Examples of the resulting reaction products are shown above as G^{LL10} and G^{LL11}.

The sugar derivative is preferably derived from galactose (Gal), mannose (Man), N-acetylglucosamine (GlcNAc), glucose (Glc), N-acetylgalactosamine (GalNAc), glucuronic acid (Gcu), fucose (Fuc) and N-acetylneuraminic acid (sialic acid), preferably from the group consisting of GlcNAc, Glc, Gal and GalNAc. More preferably the sugar derivative is derived from Gal or GalNAc, and most preferably from GalNAc.

The sugar derivative also comprises a nucleoside mono- or diphosphate, such as selected from the group consisting of uridine diphosphate (UDP), guanosine diphosphate (GDP) and cytidine diphosphate (CDP). In embodiment the sugar derivative comprises UDP.

The sugar derivative may, for example, be selected from the group consisting of GalNAz-UDP, 6-AzGal-UDP, 6-AzGalNAc-UDP, 4-AzGalNAc-UDP, 6-AzGalNAc-UDP, 6-AzGlc-UDP, 6-AzGlcNAc-UDP, 2-ketoGal-UDP, 2-N-propionyl-GalNAc-UDP and 2-(but-3-yonic acid amido)-2-deoxy-galactose-UDP. Most preferably, the sugar derivative is 6-GalNAz-UDP or 4-GalNAz-UDP.

In one embodiment, the resulting molecule is Asn-297-GlcNAc-S-R wherein: S is selected from the group consisting of galactose, mannose, N-acetylglucosamine, glucose, N-acetylgalactosamine, glucuronic acid, fucose, and N-acetylneuraminic acid; R is azide; GlcNAc is N-acetylglucosamine (optionally with fucose attached to GlcNAc); and Asn-297 is the site of N-linked glycosylation) in the Fc region of the antibody. In one embodiment, S is GalNAc. The azide can then be reacted with, for example, BCN or DBCO to give the structure shown above as G^{LL10} and G^{LL11}, respectively.

With respect to cysteine conjugation, in one embodiment the cysteine is an endogenous cysteine located in the hinge region or Fc domain. In another embodiment the cysteine in an engineered cysteine introduced in the hinge region or Fc domain.

The drug loading is the average number of PBD drugs per cell binding agent, e.g. antibody.

Thus the antibody-drug conjugate compositions of the disclosure include mixtures of antibody-drug conjugate compounds where the antibody has one or more PBD drug moieties and where the drug moieties may be attached to the antibody at various amino acid residues.

In one embodiment, the average number of dimer pyrrolobenzodiazepine groups per antibody is in the range 1 to 8. In some embodiments the range is selected from 1 to 4, 2 to 4, and 1 to 3.

### Preferred PBD agents

### ADCxAXL

ADCxAXL has the chemical structure:
Ab - (L-D)ₚ wherein L-D is 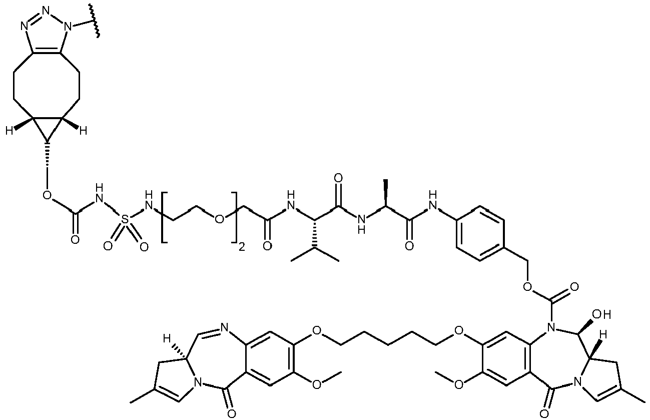 and Ab is an antibody that binds to AXL, the antibody comprising:
(a) a heavy chain having the sequence according to SEQ ID NO. 1;
(b) a light chain having the sequence according to SEQ ID NO. 2.

It is noted that "having the sequence" has the same meaning as "comprising the sequence"; in particular, in some embodiments the heavy chain of ADCxAXL is expressed with an additional terminal 'K' residue (so, ending ...SPG**K**), with the terminal K being optionally removed post-translationally to improve the homogeneity of the final therapeutic ADC product.

L-D may be conjugated to the antibody through the sidechain of the asparagine at position 302 of SEQ ID NO. 19 (for example via GlcNAc-GalNAc using the GlycoConnect approach). p may be up to 2, and is typically greater than 1.9.

### ADCxDLK1

*ADCxDLK1* has the chemical structure shown above for ADCxAXL, except that in *ADCxDLK1* "Ab" represents an antibody that binds to DLK-1, the antibody comprising:
(a) a heavy chain having the sequence according to SEQ ID NO. 11;
(b) a light chain having the sequence according to SEQ ID NO. 12.

L-D may be conjugated to the antibody through the sidechain of the asparagine at position 302 of SEQ ID NO. 13 (for example via GlcNAc-GalNAc using the GlycoConnect approach). p may be up to 2, and is typically greater than 1.9.

In particularly preferred embodiments, the PBD agent is ADCT-601 or ADCT-701.

### Formulations

The ADCs described herein can be combined with various excipients and diluents to form a pharmaceutical composition. Such excipients include buffering agents, surfactants and stabilizers/lyoprotectants such as sugars.

### Buffering Agents

The pH of the pharmaceutical compositions disclosed herein is at least about 6.2, such as from about 6.2 to about 7.0, from about 6.3 to about 6.9, about 6.4 to about 6.7, about 6.5 to 6.7, about 6.55 to 6.65, such as about 6.6.

The buffering agent is selected that can maintain the pH in the desired range i.e. has a suitable pKa and buffer range. Non-limiting examples of buffering agents include histidine (pKa 6.04, range 5.5-7.4), citrate (pKa 6.4, range 5.5-7.2), bicarbonate (pKa 6.35, range 6.0-8.0), phosphate (pKa 7.2, range 5.8-8.0) and 1,4-piperazinediethanesulphonic acid (PIPES) (pKa 6.76, range 6.1-7.5). Combinations of buffering agents may be used.

In exemplary embodiments of the present disclosure, the buffering agent is a histidine buffer, such as histidine/histidine HCI.

In some embodiments, the buffering agent is at a concentration of from about 10 mM to about 200 mM, such as at least about 10mM and equal to or less than 175 mM or 150 mM or 125 mM or 100 mM, or 50 mM.

In an exemplary embodiment of the present disclosure, the histidine buffer is at a concentration of about 10 to 30 mM, such as 15 to 25 mM, such about 20 mM.

### Surfactants

The pharmaceutical compositions disclosed here may comprise one or more surfactants. In certain embodiments, the surfactant is polysorbate (for example, polysorbate 20 and, polysorbate 80); poloxamer (e.g. poloxamer 188); N-octyl-[i-D glucopyranoside (OG); Triton; sodium dodecyl sulfate (SDS); sodium laurel sulfate; sodium octyl glycoside; lauryl-, myristyl-, linoleyl-, or stearyl-sulfobetaine; lauryl-, myristyl-, linoleyl- or stearyl- sarcosine; linoleyl-, myristyl-, or cetyl-betaine; lauroamidopropyl, cocamidopropyl-, linoleamidopropyl-, myristamidopropyl-, palmidopropyl-, or isostearamidopropylbetaine (e.g. lauroamidopropyl); myristamidopropyl-, palmidopropyl-, or isostearamidopropyldimethylamine; sodium methyl cocoyl-, or disodium methyl oleyl-taurate; polyethyl glycol, polypropyl glycol, a copolymer of ethylene and propylene glycol, such as Pluronics, or PF68; or any combination thereof.

In some embodiments, the surfactant is non-ionic. In certain exemplary embodiments, the surfactant is chosen from the group consisting of polysorbate 20 (PS20), polysorbate 80 (PS80), poloxamer 188 (P188), N-octyl-[i-D glucopyranoside (OG), and a combination thereof. In a specific embodiment, the surfactant is PS20. In another embodiment, the surfactant herein is PS80.

In certain embodiments, the surfactant (e.g., PS20) in the pharmaceutical composition is at a concentration of from about 0.01% w/v to about 0.20% w/v, such as from about 0.01% w/v to about 0.1% w/v. In certain embodiments, the surfactant (e.g., PS20) in the pharmaceutical composition is at a concentration of at least about 0.01% w/v (i.e., 0.1 mg/ml).

### Stabilizers/Lyoprotectants

The pharmaceutical compositions disclosed here may comprise one or more stabilizers/lyoprotectants, typically a sugar. Without wishing to be bound by theory, a sugar functions to promote cryoprotection of the pharmaceutical composition, thereby preventing aggregation and maintaining chemical and physical stability of the pharmaceutical composition. In certain embodiments, the sugar is glucose, fructose, sucrose, trehalose, arginin, glycerin, prolin, dextran, dextran 40, glycerol, mannitol, or sorbitol.

In one embodiment, the stabilizer/lyoprotectant, is sucrose, such as where the only sugar present is sucrose.

The stabilizer/lyoprotectant, such as sucrose, may be at a concentration of from about 50 mM to 300 mM, such as about 50 mM to about 250 mM, about 100 mM to about 200 mM, about 150 mM to about 200 mM or about 160 mM to about 180 mM. In a specific embodiment, the buffering agent is at a concentration of about 150 mM, 160 mM, 165 mM, 170 mM, 175 mM, 180 mM, 190 mM, or 200 mM. In some embodiments, the sugar is at a concentration of 150 mM to 200 mM.

In an exemplary embodiment of the present disclosure, sucrose is present at a concentration of about 175 mM.

The concentration of the ADC in the formulation is typically from about 5 mg/ml to about 60 mg/ml, about 5 mg/ml to about 50 mg/ml, about 5 mg/ml to about 40 mg/ml, about 5 mg/ml to about 30 mg/ml, or about 5 mg/ml to about 20 mg/ml. In one embodiment the concentration of the ADC is about 5 mg/ml. Where the formulation is an intermediate, for example during manufacturing steps including purification steps, the concentration may be higher than in the final product. For example the concentration may be at least about 20 mg/ml, such as from about 20 mg/ml to about 80 mg/ml or at least 30 mg/ml or 40 mg/ml, such as from about 30 mg/ml to about 80 mg/ml.

The pharmaceutical composition may be in the form of a liquid composition or a lyophilized formulation. The various parameters provided about in relation to the amounts of the ingredients are based on a liquid formulation. A liquid formulation may be an intermediate in the preparation of the final drug product, or may be the final drug product itself. A liquid formulation may also be the result of reconstitution of a lyophilized formula, e.g. in a vial, prior to administration.

Lyophilized formulations can be prepared by methods known in the art. A "lyophilized" composition as used herein refers to a liquid composition that has been subjected to freeze-drying via the process of lyophilization, resulting in a "cake". Accordingly, the compositions of the invention in liquid form, such as resulting from the manufacturing process, may be subject to a freeze-drying step to produce a composition in lyophilized form.

### Stability

In some embodiments, the physical stability, chemical stability, or biological activity of the pharmaceutical composition in a liquid state or a lyophilized form is evaluated or measured. Any methods known in the art may be used to evaluate the stability and biological activity of the pharmaceutical composition of the present disclosure. In some embodiments, stable pharmaceutical composition (or formulation) is one in which the ADC therein essentially retains its physical stability and/or chemical stability and/or biological activity upon storage;, e.g., storage for a specified period or duration of time (such as hours, days, months, years, etc.) under specified conditions (such as temperature, relative humidity, residual moisture, presence or absence of light, following a period of agitation, etc.).

In some embodiments, the ADC present in the pharmaceutical composition is biologically stable (e.g., retains its biological activity) if the biological activity of the ADC (especially that of the PBD warhead) at a given time is within the range accepted by a national or regional regulatory agency for pharmaceutical products (e.g., the Federal Drug Administration (FDA) of the United States, the Therapeutic Goods Administration (TGA) of Australia, the European Medicines Agency (EMA) of the European Union, etc.). For example, the biological activity of an ADC or a pharmaceutical composition comprising such an immunoconjugate can be measured by its ability to bind antigen. Additionally or alternatively, the biological activity of an ADC is measured by its ability to inhibit cell growth and/or cell proliferation, e.g., *in vitro or in vivo,* or the ability to induce cell death, including programmed cell death (apoptosis) e.g., *in vitro or in vivo.* Antigen binding assays and *in vitro* cytotoxicity assays (with H1299 cells) are described in WO2018/146189, the contents of which are here incorporated by reference.

In one embodiment "stable" means retaining at least 90%, 80% or 70% biological activity as determined by an *in vitro* cytotoxicity assay.

The intact PBD dimer conjugate and other associated degraded forms absorb at 340 nm. Thus, the stability of the PBD moiety may also be determined, as in the Examples, by UV analysis in combination with MS peptide identification. Reduced peptide mapping was determined to be the most critical assay as it was successfully able to track the degradation of the conjugate related peptides into the aromatized form which was shown to be less potent (oxidation in the C ring - see the structure in the Examples). In one embodiment, after storage at 14 days at 25°C, the percentage of intact conjugate (e.g. as the relative area of the peaks in peptide mapping) is greater than 35%, 40%, 50%, 60%, 70%, 80% or 90% and/or the percentage of aromatized variant is less than 65%, 60%, 50%, 40%, 30%, 20%, 10% (e.g. as the relative area of the peaks in peptide mapping).

In some embodiments, the pharmaceutical composition of the present disclosure, such as a lyophilized formulation, is stable at -20°C for at least about 6 months, at least about 8 months, at least about 10 months, at least about 12 months, at least about 14 months, at least about 16 months, at least about 18 months, at least about 20 months, at least about 24 months, or at least about 3 years, when protected from light.

In some embodiments, the pharmaceutical composition of the present disclosure, such as a liquid formulation or lyophilized formulation is stable at 2°C to 8°C (e.g., 5°C ± 3°C) for at least about 6 months, at least about 8 months, at least about 10 months, at least about 12 months, at least about 14 months, at least about 16 months, at least about 18 months, at least about 20 months or at least about 24 months, when protected from light.

In some embodiments, the pharmaceutical composition of the present disclosure, such as a reconstituted liquid formulation, is stable at 25°C for at least about 12 hours, at least about 14 hours, at least about 16 hours, at least about 18 hours, at least about 20 hours, at least about 22 hours, or at least about 1 day, 2 days or 3 days.

### Dosage forms and Reconstitution for administration

The present disclosure provides pharmaceutical compositions and liquid compositions suitable for intravenous (IV) administrations. The compositions to be used for *in vivo* IV administration should be sterile. This is readily accomplished by filtration through sterile filtration membranes, prior to, or following, preparation of the pharmaceutical composition. In certain embodiments, the pharmaceutical composition of the present disclosure is reconstituted from a lyophilized formulation with sterile water for injection (SWFI). In some embodiments, the reconstituted composition is further diluted into an isotonic buffer in an intravenous (IV) bag.

Thus, in one aspect, the present disclosure provides a liquid pharmaceutical composition including an ADC, such as ADCxAXL, as described herein, which has been reconstituted from a lyophilized formulation as described herein, in a suitable diluent such as water. In one embodiment the liquid formulation has a pH of from about 6.3 to 6.7, such as about 6.4 to 6.6; and comprises histidine at a concentration of from about 10 to 50 mM, such as about 20 mM; polysorbate 20 at a concentration of from about 0.01 to 0.05% w/v, such as about 20 mM; and a sugar, such as sucrose, at a concentration of from about 150 to 200 mM, such as about 175 mM. The liquid formulation comprises the ADC at a concentration of from about 5 mg/ml to 20 mg/ml, such as about 5 mg/ml.

The composition of the invention may be presented in a number of dosage forms known in the art. For example a liquid formulation may be in the form of a pre-filled syringe or a vial; a lyophilized formulation may be in the form of a vial, such as a glass vial; or an IV bag containing the pharmaceutical composition or liquid composition of the present disclosure.

### Therapeutic Uses

The pharmaceutical compositions of the present invention may be administered to a subject in need of treatment, such as a patient suffering from a proliferative disorder e.g. characterized by the overexpression of a tumor antigen. An exemplary proliferative disorder is cancer. Examples of cancer include, but are not limited to, hematopoietic cancers or blood-related cancers, such as lymphoma, leukemia, myeloma or lymphoid malignancies, but also solid cancers/tumors, such as cancers of the spleen and cancers of the lymph nodes and also carcinoma, blastoma and sarcoma.

### Sequence listing part of the description

### AXL

SEQ ID NO. 1 [1H12 VH] 
QVQLVESGGGVVQPGRSLRLSCAASGFTFSSYGMSWVRQAPGKGLEWVATISSGGSYTYYPDSVKGRFTI
SRDNSKNTLYLQMNSLRAEDTAVYYCARHPIYYTYDDTMDYWGQGTTVTVSS
 SEQ ID NO. 2 [1H12 VL] 
EIVLTQSPGTLSLSPGERATLSCSASSSVSSGNFHWYQQKPGLAPRLLIYRTSNLASGIPARFSGSGSGT
DFTLTISSLEPEDFAVYYCQQWSGYPWTFGGGTKLEIK
 SEQ ID NO.3 [1H12 Heavy Chain] 
QVQLVESGGGVVQPGRSLRLSCAASGFTFSSYGMSWVRQAPGKGLEWVATISSGGSYTYYPDSVKGRFTI

SRDNSKNTLYLQMNSLRAEDTAVYYCARHPIYYTYDDTMDYWGQGTTVTVSSASTKGPSVFPLAPSSKST

SGGTAALGCLVKDYFPEPVTVSWNSGALTSGVHTFPAVLQSSGLYSLSSVVTVPSSSLGTQTYICNVNHK

PSNTKVDKKVEPKSCDKTHTCPPCPAPELLGGPSVFLFPPKPKDTLMISRTPEVTCVVVDVSHEDPEVKF

NWYVDGVEVHNAKTKPREEQYNSTYRVVSVLTVLHQDWLNGKEYKCKVSNKALPAPIEKTISKAKGQPRE

PQVYTLPPSREEMTKNQVSLTCLVKGFYPSDIAVEWESNGQPENNYKTTPPVLDSDGSFFLYSKLTVDKS

RWQQGNVFSCSVMHEALHNHYTQKSLSLSPG
 SEQ ID NO.4 [1H12 Light Chain]

EIVLTQSPGTLSLSPGERATLSCSASSSVSSGNFHWYQQKPGLAPRLLIYRTSNLASGIPARFSGSGSGT
DFTLTISSLEPEDFAVYYCQQWSGYPWTFGGGTKLEIKRTVAAPSVFIFPPSDEQLKSGTASVVCLLNNF
YPREAKVQWKVDNALQSGNSQESVTEQDSKDSTYSLSSTLTLSKADYEKHKVYACEVTHQGLSSPVTKSF
NRGEC

| | |
|---|---|
| SYGMS | SEQ ID NO.5 [1H12 VH CDR1] |
| TISSGGSYTYYPDSVKG | SEQ ID NO.6 [1H12 VH CDR2] |
| HPIYYTYDDTMDY | SEQ ID NO.7 [1H12 VH CDR3] |
| SASSSVSSGNFH | SEQ ID NO.8 [1H12 VL CDR1] |
| RTSNLAS | SEQ ID NO.9 [1H12 VL CDR2] |
| QQWSGYPWT | SEQ ID NO.10 [1H12 VL CDR3] |

### DLK-1

SEQ ID NO.11 [HuBa-1-3d VH] 
QVQLVQSGAEVKKPGASVKVSCKGSGYTFTDYAMHWVRQAPGQGLEWIGVISTYYGNTNYNQKFKGKATM
TVDKSTSTAYMELRSLRSDDTAVYYCARGGLREYYYAMDYWGQGTMVTVSS
 SEQ ID NO.12 [HuBa-1-3d VL] 
DIVMTQSPDSLAVSLGERATINCKSSQSLLNSSNQKNYLAWYQQKPGQPPKLLVYFASTRESGVPDRFSG

SGSGTDFTLTISSLQAEDVAVYYCQQHYSTPPTFGQGTKLEIK
 SEQ ID NO.13 [HuBa-1-3d Heavy Chain] 
QVQLVQSGAEVKKPGASVKVSCKGSGYTFTDYAMHWVRQAPGQGLEWIGVISTYYGNTNYNQKFKGKATM
TVDKSTSTAYMELRSLRSDDTAVYYCARGGLREYYYAMDYWGQGTMVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDSDGSFFLYSKLTVDKSR
WQQGNVFSCSVMHEALHNHYTQKSLSLSPG
 SEQ ID NO.14 [HuBa-1-3d Light Chain]

DIVMTQSPDSLAVSLGERATINCKSSQSLLNSSNQKNYLAWYQQKPGQPPKLLVYFASTRESGVPDRFSG
SGSGTDFTLTISSLQAEDVAVYYCQQHYSTPPTFGQGTKLEIKRTVAAPSVFIFPPSDEQLKSGTASVVC
LLNNFYPREAKVQWKVDNALQSGNSQESVTEQDSKDSTYSLSSTLTLSKADYEKHKVYACEVTHQGLSSP
VTKSFNRGEC

| | |
|---|---|
| DYAMH | SEQ ID NO.15 [HuBa-1-3d VH CDR1] |
| VISTYYGNTNYNQKFKG | SEQ ID NO.16 [HuBa-1-3d VH CDR2] |
| GGLREYYYAMDY | SEQ ID NO.17 [HuBa-1-3d VH CDR3] |
| KSSQSLLNSSNQKNYLA | SEQ ID NO.18 [HuBa-1-3d VL CDR1] |
| FASTRES | SEQ ID NO.19 [HuBa-1-3d VL CDR2] |
| QQHYSTPPT | SEQ ID NO.20 [HuBa-1-3d VL CDR3] |

SEQ ID NO.21 [HuBa-1-3d Heavy Chain, terminal K]

QVQLVQSGAEVKKPGASVKVSCKGSGYTFTDYAMHWVRQAPGQGLEWIGVISTYYGNTNYNQKFKGKATM
TVDKSTSTAYMELRSLRSDDTAVYYCARGGLREYYYAMDYWGQGTMVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDSDGSFFLYSKLTVDKSR
WQQGNVFSCSVMHEALHNHYTQKSLSLSPGK

Some aspects and embodiments of the disclosure are described below in more detail with reference to the following examples, which are illustrative only and non-limiting. The examples refer to the following figure:

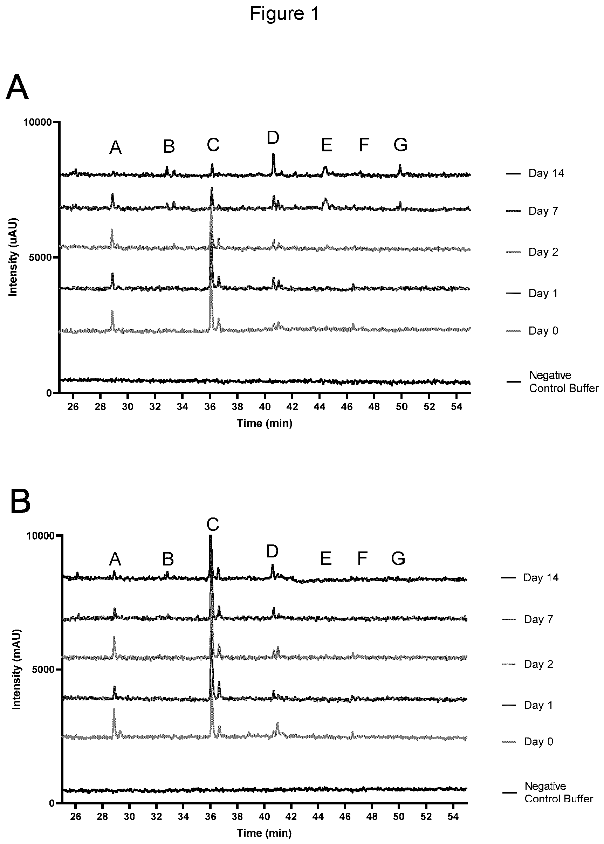

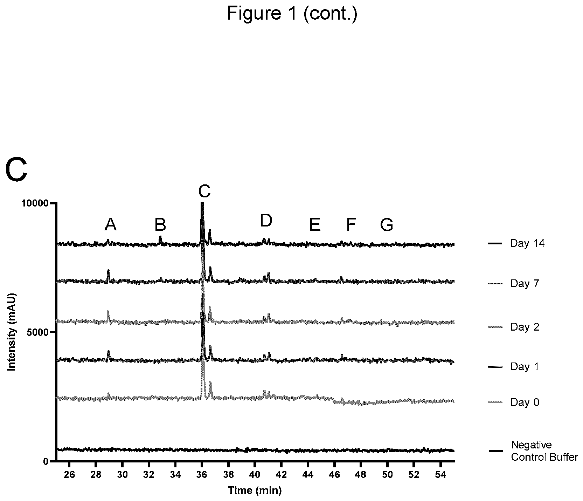

Figure 1 - Offset zoom scale (25-55 minutes) A340nm chromatograms of pH screen samples over the two-week time course with labelled peaks: A) pH 6.0, B) pH 7.0, C) pH 8.0.

### EXAMPLES

### Introduction

A liquid drug product for ADCxAXL provided as a 5 mg/mL formulation in 30 mM histidine/histidine HCI, 0.02% (w/v) PS20 at pH 6.0 was initially developed. The liquid formulation is stable at its intended frozen (≤ -60°C) storage condition. However, we observed a decrease in the cell-based potency assay at room temperature. It was determined that this was due to aromatization within the SG3199 PBD molecule, as shown below, resulting from oxidation in the C ring:

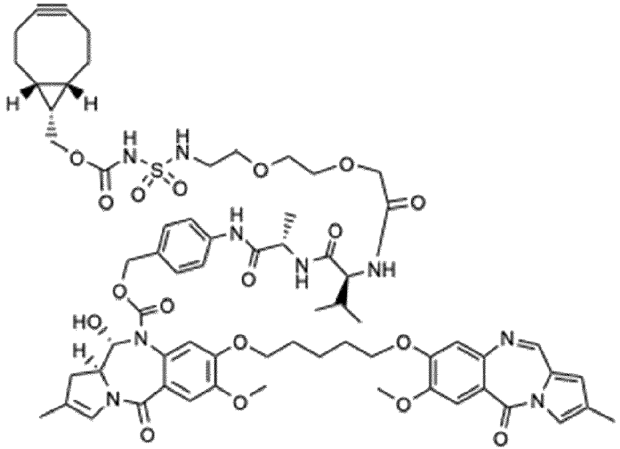

Formulation studies were performed to mitigate the decrease in potency and improve product stability during manufacturing and use.

### pH Screening

The purpose of this study was to understand the effect of pH on product stability. A pH dependent potency change was observed where at lower pH PBD-dimer toxin degraded into an aromatized variant at an increased rate as indicated by peptide mapping. The degradation rate of PBD-dimer toxin was found to be correlated with the rate of decrease in the potency assay.

The study evaluated pH effect ranging from 6.0 to 8.0 using a poly buffer system. ADCxAXL was buffer exchanged into five buffers with pH specifications of 6.0, 6.5, 7.0, 7.5, and 8.0. All buffers contained 10 mM citrate and 10 mM phosphate to ensure buffering capacity across the desired pH range and to isolate the effect of pH. All samples were placed on stability at room temperature for 2 weeks.

Reduced peptide mapping was determined to be the most critical assay as it was successfully able to track the degradation of the conjugate related peptides into the aromatized form which was shown to be less potent and demonstrated a similar potency loss trend for stability samples at pH 6. The results showed samples at high pH were significantly more stable than samples at lower pH, and there was a clear trend of increasing stability with increasing pH. Samples at high pH did show increased levels of PTMs such as deamidation, but the amount detected is still considered relatively low since the time of exposure at room temperature for manufacturing and clinical use is around 2-3 days.

The intact PBD dimer conjugate and other associated degraded forms absorb at 340 nm. UV analysis in combination with MS peptide identification was used to assess conjugate degradation in the different formulations. Offset zoom scale A340 nm chromatograms (with labelled peaks) of the pH 6, 7 and 8 formulations are shown in Figure 1 (A, B and C respectively) with the associated labelled peak identities (peptide and modification) in Table 1. At pH 6, the conjugate more readily degrades into the aromatized form (the most abundant variant), as seen by the disappearance of peak C and growth of peaks B and D. Conjugate degradation into the aromatized form is minimized with the increasing pH.

**Table 1 - A340nm Conjugated Peptide Peak Identities**

| **Peak Label** | **Retention Time (min)** | **Peptide** | **Modification** |
|---|---|---|---|
| A | 28-30 | TKPREEQYNSTYR | Intact Conjugate |
| B | 32-34 | TKPREEQYNSTYR | Aromatized Variant |
| C | 36 | EEQYNSTYR | Intact Conjugate |
| C | 36.5 | TKPREEQYNSTYRVVSVLTVLHQDWLNGK | Intact Conjugate |
| D | 40-42 | EEQYNSTYR | Aromatized Variant |
| E | 44-46 | TKPREEQYNSTYRVVSVLTVLHQDWLNGK | Aromatized Variant |
| F | 46-48 | EEQYNSTYRVVSVLTVLHQDWLNGK | Intact Conjugate |
| G | 49-51 | EEQYNSTYRVVSVLTVLHQDWLNGK | Aromatized Variant |

### The relative abundance of the intact conjugate and the aromatized variant are listed in Table 2

Table 2 - Relative Area Percentages of Intact Conjugate and aromatized Variant in pH Screen Samples. The decrease in intact conjugate and increase in the aromatized form aligns well, confirming the aromatized form is the main form of conjugate degradation. The relative abundance of representative monoclonal antibody PTMs (Aspartate isomerization, Methionine oxidation, and Asparagine deamidation) for formulations pH 6, 7, and 8 are listed in Table 3. While the level of deamidation increases across the time course for the higher pH formulation, the overall level is still relatively low with an increase of less than one percent over the expected manufacturing hold times of 2 days. Deamidation is expected at higher pH antibody formulations. The levels of oxidation and isomerization did not change significantly across formulation pH or the time course. All three representative PTM's were tracked in future phases of the formulation development and monitored to ensure the levels were acceptable and not correlating with formulation components.

**Table 2 - Relative Area Percentages of Intact Conjugate and aromatized Variant in pH Screen Samples**

| **pH** | **Time point (days)** | **Relative Area Percentage (%)** | |
|---|---|---|---|
| | | **Intact Conjugate** | **-2 Da cyclized variant** |
| 6.0 | 0 | 75.1 | 9.2 |
| | 1 | 68.5 | 15.8 |
| | 2 | 61.4 | 22.7 |
| | 7 | 32.3 | 53.5 |
| | 14 | 13.2 | 70.3 |
| 6.5 | 0 | 73.9 | 11.9 |
| | 1 | 70.7 | 15.5 |
| | 2 | 67.3 | 19.7 |
| | 7 | 51.8 | 36.2 |
| | 14 | 40.2 | 48.7 |
| 7.0 | 0 | 78.2 | 11.7 |
| | 1 | 74.4 | 12.0 |
| | 2 | 74.8 | 14.3 |
| | 7 | 68.4 | 19.2 |
| | 14 | 62.3 | 26.5 |
| 7.5 | 0 | 77.8 | 11.4 |
| | 1 | 78.2 | 11.9 |
| | 2 | 76.4 | 12.0 |
| | 7 | 74.4 | 13.8 |
| | 14 | 73.2 | 16.2 |
| 8.0 | 0 | 76.1 | 11.2 |
| | 1 | 77.8 | 11.0 |
| | 2 | 76.9 | 11.3 |
| | 7 | 77.1 | 11.7 |
| | 14 | 75.8 | 11.9 |

**Table 3 - Relative Area Percentages of Representative PTM's**

| **PTM** | **Formulation pH** | **Relative Area Percentage** (%) | | | | |
|---|---|---|---|---|---|---|
| | | **Day 0** | **Day 1** | **Day 2** | **Day 4** | **Day 14** |
| D106 Isomerization | 6 | 0.69 | 0.73 | 0.77 | 1.03 | 1.31 |
| | 7 | 0.74 | 0.77 | 0.78 | 0.91 | 1.16 |
| | 8 | 0.74 | 0.81 | 0.85 | 1.02 | 1.29 |
| M257 Oxidation | 6 | 3.71 | 3.79 | 3.90 | 4.38 | 4.50 |
| | 7 | 3.40 | 3.54 | 3.46 | 4.03 | 4.60 |
| | 8 | 3.89 | 3.68 | 4.12 | 3.79 | 4.59 |
| N394 Deamidation | 6 | 1.50 | 1.43 | 1.55 | 1.63 | 1.56 |
| | 7 | 1.62 | 1.56 | 1.72 | 1.88 | 2.14 |
| | 8 | 1.63 | 1.82 | 2.08 | 3.56 | 5.30 |

### pH/Buffer and Excipients Screening

A narrower pH range of pH 6.5 to pH 7.75 with different buffer systems and excipients was further evaluated for product stability. Sucrose, a lyoprotectant and cryoprotectant was also included and evaluated.

Samples were buffer exchanged into four formulations containing 175 mM sucrose, a component for lyophilization as the lyoprotectant, and 20 mM histidine at pH 6.5, 20 mM sodium phosphate at pH 7.0, and 20 mM Tris at pH 7.5 and 7.75. Formulation components and labels are noted in Table 4.

**Table 4 - Labels and components for all formulations tested**

| **Formulation Label** | **Formulation Components** |
|---|---|
| Formulation 1 | 20 mM Histidine, 175 mM Sucrose, pH 6.5 |
| Formulation 2 | 20 mM Sodium Phosphate, 175 mM Sucrose, pH 7.0 |
| Formulation 3 | 20 mM Tris, 175 mM Sucrose, pH 7.5 |
| Formulation 4 | 20 mM Tris, 175 mM Sucrose, pH 7.75 |

During the buffer exchange, using centrifugal filters, concentration measurement of the samples after centrifugation/concentration showed that formulation 1 achieved a concentration three times higher than formulations 2-4. Samples were buffer exchanged into the above buffers using Amicon Ultra-4 10 kDa MWCO centrifugal filters. ADCxAXL DS at 6.25 mg/mL was then added to each of the filters and the samples were spun at 3500 RCF for 30 minutes to reduce the volume to 10% of the original volume. After 3 buffer exchanges, the concentration and appearance of the solutions was evaluated.

During the concentration step, it was observed that samples in the phosphate and tris buffers were not decreasing in volume at the same rate as the sample in the histidine buffer. Samples were additionally assessed for visible particles. Visual assessment of the samples determined that the formulations at pH 7.0 - 7.75 contained large particles in solution that appeared to be fibrous in nature. The particle descriptions were recorded but were not investigated further. The results in Table 5 show that ADCxAXL has a higher solubility in histidine (Formulation 1) than in phosphate or tris (Formulations 2-4). The solubility was further explored with additional excipients, such as arginine, to increase solubility but was not successful.

**Table 5 - Concentrations after centrifugation at 3500 RCF for 30 minutes and visible particle assessment results**

| **Formulation Label** | **Concentration (mg/mL)** | **Visible Particle Assessment** |
|---|---|---|
| Formulation 1 (pH 6.5) | 69.2 | No |
| Formulation 2 (pH 7.0) | 25.0 | Yes - Few larger particles, appear to be fibrous in nature |
| Formulation 3 (pH 7.5) | 20.6 | Yes - Few larger particles, appear to be fibrous in nature |
| Formulation 4 (pH 7.75) | 23.7 | Yes - Few larger particles, appear to be fibrous in nature |

Furthermore, buffer exchanged formulated samples were then split equally into 4 time points and incubated at 25°C with time points to be pulled after 0, 4, 7, and 14 days. Incubation at 25°C was selected to investigate the degradation that may occur during the manufacturing process. After each time point was pulled, the samples were sub aliquoted into method specific aliquots and stored at -80°C until analysis.

Reduced peptide map showed similar results as seen during the pH screen, samples at higher pH were more stable with lower amount of aromatized form. The conjugate peak labels are listed in Error! **Reference source not found..** There is a clear trend seen with less degradation of the conjugate occurring in formulations 2 - 4 and an increase in degradation rate into the aromatized form with decreasing pH. Although the toxin is significantly more stable at pH 7.5 or 7.75 with little to no degradation, the level of toxin degradation at pH 6.5 was significantly lower than that seen at pH 6.0. At pH 6.5 there is a decrease in toxin degradation to ~ 1% per day and no potential issues during the concentration or diafiltration steps required during processing, which is an advantage over Formulations 2 to 4.

Thus, of the formulations tested, Formulation 1 was determined to be the optimum pH at 6.5 because the product is more soluble in histidine at pH 6.5 than at higher pH targets, maintains stability at 40°C when assessed by SEC and icIEF and the conjugate maintains sufficient stability at 25°C over 14 days when analyzed by reduced peptide map.

**Table 6 - Relative Area Percentages of intact conjugate and aromatized variant**

| **Incubation Temperature (°C)** | **Formulation** | **Time point days** | **Relative Area Percentages (%)** | |
|---|---|---|---|---|
| | | | **Intact Conjugate** | **Aromatized Variant** |
| 25°C | Formulation 1 (pH 6.5) | 0 | 79.3 | 19.8 |
| | | 4 | 72.7 | 26.9 |
| | | 10 | 59.4 | 40.0 |
| | | 14 | 60.0 | 39.8 |
| | Formulation 2 (pH 7.0) | 0 | 81.7 | 17.8 |
| | | 4 | 77.8 | 22.6 |
| | | 10 | 73.4 | 27.0 |
| | | 14 | 71.1 | 29.5 |
| | Formulation 3 (pH 7.5) | 0 | 82.3 | 18.4 |
| | | 4 | 80.1 | 20.9 |
| | | 10 | 78.6 | 21.8 |
| | | 14 | 79.4 | 21.7 |
| | Formulation 4 (pH 7.75) | 0 | 79.9 | 20.9 |
| | | 4 | 81.0 | 18.8 |
| | | 10 | 80.6 | 19.0 |
| | | 14 | 79.3 | 19.8 |

### Conclusions

Formulation pH was determined to be the most critical component for both conjugate stability and product solubility. Higher pH maintains stability of the conjugate portion of the molecule, while formulations with lower pH increase sample solubility significantly, which is important for manufacturing. Overall, based on the product stability and solubility, histidine buffer at pH 6.3-6.9 was shown to have the best properties: the final formulation exhibited improved stability of the drug-linker and sufficient product solubility for manufacturing. The new formulation in both liquid and lyophilized forms mitigates the potency decrease observed in the original pH 6.0 formulation.

A number of publications are cited above to more fully describe and disclose the disclosures. The entirety of each of the references mentioned in this disclosure are hereby is incorporated by reference. Features and specific embodiments in individual sections apply to other sections mutatis mutandis.

## Claims

1. A pharmaceutical composition comprising an antibody drug conjugate (ADC) of formula:
Ab-(L-D)ₚ
wherein:
Ab is an antibody;
(L-D) is 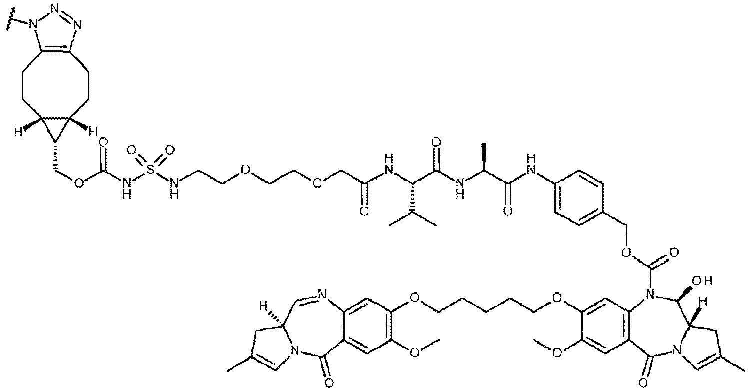 and p is a value from about 1 to about 8;
wherein the pharmaceutical composition has a pH of at least about 6.2.

2. The pharmaceutical composition of claim 1 which has a pH of from about 6.2 to about 7.0, such as from about 6.3 to about 6.9

3. The pharmaceutical composition of claim 2 which has a pH of from about 6.4 to about 6.7.

4. The pharmaceutical composition of any one of the preceding claims, which comprises a buffering agent.

5. The pharmaceutical composition of claim 4 wherein the buffering agent includes histidine.

6. The pharmaceutical composition of claim 5 wherein the histidine is at a concentration of from about 10 to about 30 mM, such as about 20 mM.

7. The pharmaceutical composition of any one of the preceding claims which comprises a surfactant.

8. The pharmaceutical composition of claim 7 wherein the surfactant is polysorbate 20.

9. The pharmaceutical composition of claim 7 or claim 8 wherein the surfactant is at a concentration of from about 0.01 to 0.1% w/v.

10. The pharmaceutical composition of any one of the preceding claims further comprising a sugar.

11. The pharmaceutical composition of claim 10 wherein the sugar is at a concentration of from about 100 mM to about 250 mM, such as about 175 mM.

12. The pharmaceutical composition of any one of the preceding claims wherein the ADC is at a concentration of about 5 mg/ml to about 60 mg/ml, about 5 mg/ml to about 50 mg/ml, about 5 mg/ml to about 40 mg/ml, about 5 mg/ml to about 5 mg/ml, or about 5 mg/ml to about 20 mg/ml.

13. The pharmaceutical composition of any one of the preceding claims wherein Ab is an anti-AXL antibody which comprises a heavy chain and a light chain, wherein (i) the heavy chain comprises a VH domain having a VH CDR1 with the amino acid sequence of SEQ ID NO.3; a VH CDR2 with the amino acid sequence of SEQ ID NO.4; and a VH CDR3 with the amino acid sequence of SEQ ID NO.5; and (ii) the light chain comprises a VL domain having a VL CDR1 with the amino acid sequence of SEQ ID NO.6; a VL CDR2 with the amino acid sequence of SEQ ID NO.7, and a VL CDR3 with the amino acid sequence of SEQ ID NO.8.

14. A lyophilized pharmaceutical composition produced by lyophilization of a pharmaceutical composition according to any one of claims 1 to 13 in liquid form.

15. The pharmaceutical composition of any one of claims 1 to 13, wherein the pharmaceutical composition of claim 15 is reconstituted with sterile water for injection (SWFI) to produce a reconstituted composition.
